# EUROPÄISCHE PATENTSCHRIFT

(11) **EP 2 015 676 B1**
(45) Veröffentlichungstag und Bekanntmachung des Hinweises auf die Patenterteilung: **29.12.2010**
(21) Anmeldenummer: 07727080.9
(22) Anmeldetag: 20.03.2007
(51) Int. Cl.: A61B 5/103

(54) **INTERFEROMETRISCHE MESSVORRICHTUNG ZUR OPTISCHEN MESSUNG AN HAUTSTRUKTUREN**
INTERFEROMETRIC MEASURING INSTRUMENT FOR TAKING OPTICAL MEASUREMENTS ON SKIN STRUCTURES
DISPOSITIF DE MESURE INTERFÉROMÉTRIQUE POUR LA MESURE OPTIQUE AU NIVEAU DE STRUCTURES CUTANÉES

(30) Priorität: 04.05.2006 DE 102006020720
(43) Veröffentlichungstag der Anmeldung: 21.01.2009
(73) Patentinhaber: Robert Bosch GmbH, 70442 Stuttgart (DE)
(72) Erfinder: KALLMANN, Ulrich, 72076 Tuebingen (DE)
(86) Internationale Anmeldenummer: PCT/EP2007/052603
(87) Internationale Veröffentlichungsnummer: WO 2007/128614

(56) Entgegenhaltungen:
- DE-A1- 19 540 456
- US-A1- 2004 100 376
- US-A1- 2004 254 474
- US-A1- 2005 271 547
- US-B1- 6 501 551
- US-B1- 6 725 073
- HUAFENG DING ET AL: "Refractive indices of human skin tissues at eight wavelengths and estimated dispersion relations between 300 and 1600 nm; Refractive indices of human skin tissues and estimated dispersion relations" PHYSICS IN MEDICINE AND BIOLOGY, INSTITUTE OF PHYSICS PUBLISHING, BRISTOL, GB, Bd. 51, Nr. 6, 21. März 2006 (2006-03-21), Seiten 1479-1489, XP020096187 ISSN: 0031-9155

## Beschreibung

Die Erfindung bezieht sich auf eine interferometrische Messvorrichtung und ein interferometrisches Messverfahren zur optischen Messung an Hautstrukturen gemäß dem Oberbegriff des Anspruchs 1.

Eine interferometrische Messvorrichtung bzw. ein interferometrisches Messverfahren dieser Art ist in der US 6 725 073 B1 gezeigt. Bei dieser bekannten Messvorrichtung bzw. diesem bekannten Verfahren wird eine tiefenabhängige Abnahme des Streulichts mittels einer interferometrischen Anordnung bestimmt, wobei in der Hautstruktur ein optisches Element mit reflektierenden Schichten implantiert ist.

Weitere optische Messvorrichtungen bzw. Messverfahren sind in der US 2005/0271547 A1 und der US 6 501 551 B1 gezeigt.

Eine weitere interferometrische Messvorrichtung ist in R. Kuranov, D. Prough, V. Sapozhnikova, I. Cicenaite, R. Esenaliev, "In vivo application of 2-D lateral scanning mode Optical Coherence Tomography for glucose sensing", in Proc. SPIE, 6007, 60070K-1 60070K-6 (2005) angegeben. Bei dieser bekannten Messvorrichtung bzw. diesem bekannten Messverfahren wird die Blutzuckerkon-zentration mittels einer optischen Kohärenz-Tomographie (OCT) ermittelt, indem die Zuckerkonzentration in der Gewebeflüssigkeit der Haut optisch mittels der interferometrischen Messvorrichtung auf der Grundlage einer Brechungsindexänderung erfasst wird, wobei die Messung der Indexänderung indirekt über die tiefenaufgelöste Messung des in die optische Apparatur zurückgestreuten Lichts erfolgt. Der Messung liegt dabei die Erkenntnis zugrunde, dass das Ausmaß der Streuung durch den Brechungsindex der Gewebeflüssigkeit maßgeblich beeinflusst wird; die Streuintensität hängt von dem optischen Brechungsindexsprung ab, der zwischen der Gewebeflüssigkeit und Streukörpern in der Haut (z.B. Hautzellen) besteht. Ein Nachteil dieser bekannten Methode ist, dass die benötigte Messzeit in der Größenordnung einiger Minuten liegt, um durch Mittelung über eine große Anzahl von Messungen und anschließende Datennachbearbeitung zu einer genügenden Genauigkeit zu gelangen. Ursache dafür sind zum einen die optischen Verhältnisse in der Haut. Zum anderen beeinflussen die Messbedingungen am lebenden Objekt die Ergebnisse der Messung.

Der Erfindung liegt die Aufgabe zugrunde, eine möglichst schnelle und dabei zuverlässige Messvorrichtung bzw. Messmethode zur Bestimmung einer Eigenschaft der Hautstruktur, insbesondere der Gewebeflüssigkeit bereitzustellen.

### Offenbarung der Erfindung

### Vorteile der Erfindung

Diese Aufgabe wird mit den Merkmalen des Anspruches 1 gelöst. Hierbei ist vorgesehen, dass zum Zurückstreuen eines Anteils des in die Haut geleiteten Objektstrahls ein Implantat mit einer dem einfallenden Objektstrahl zugewandten Vorderseite und einer davon abgewandten Rückseite in die Haut eingebracht ist, das gegenüber der umgebenden Hautsubstanz einen unterschiedlichen Brechungsindex aufweist.

Das Implantat ermöglicht die optische Messung an dem definierten Übergang zwischen der Hautsubstanz, insbesondere also der Gewebeflüssigkeit, und dem Implantat. Dadurch werden relativ genaue und zuverlässige Messergebnisse erhalten.

Verschiedene alternative Ausgestaltungen der Messvorrichtung bzw. Vorgehensweisen bei dem Verfahren ergeben sich dadurch, dass zum Bewirken eines Tiefenscans in der Haut und damit zum Erzeugen der Intensitätsmodulation eine Scanvorrichtung vorgesehen ist, die in der Weise aufgebaut ist, dass die optische Weglänge des Referenzstrahls in dem Referenzzweig oder die Wellenlänge der Lichtquelle definiert geändert wird oder dass in der Detektorvorrichtung eine spektrale Auflösung und Auswertung erfolgt, wobei eine Umrechnung in den Ortsraum vorgenommen wird.

Zur Genauigkeit und Zuverlässigkeit der Messung tragen die Maßnahmen bei, dass das Implantat mit einer Vielzahl von Grenzflächen relativ zur Gewebeflüssigkeit zwischen seiner Vorderseite und seiner Rückseite versehen ist und dass die Auswertung in der Weise ausgelegt ist, dass bezüglich des Durchgangs des Objektstrahls durch das Implantat auf der Basis der Intensitätsmodulation im Interferenzsignal die tiefenabhängige Abnahme des Streulichts und/oder die mittlere optische Weglänge unter Bewertung eines mittleren Brechungsindex zur Bestimmung einer Eigenschaft der Hautstruktur ermittelt wird/werden.

Eine Optimierung der Messbedingungen lässt sich dadurch erreichen, dass das Implantat so geformt, strukturiert oder beschichtet ist, dass das von seiner Vorderseite herrührende Vorderseitensignal und das von seiner Rückseite herrührende Rückseitensignal besonders hervorgehoben sind.

Um Störungen in Folge einer Dispersion der Hautschichten möglichst zu unterdrücken, besteht eine weitere vorteilhafte Maßnahme darin, dass im Referenzzweig ein auf den Durchgangsabschnitt der Haut abgestimmtes dispersionskompensierendes Medium eingebracht ist.

Bei dem Verfahren besteht eine günstige Vorgehensweise bei der Auswertung darin, dass zum Bestimmen der Eigenschaft der Hautstrukturen auf der Basis der Intensitätsmodulation die tiefenabhängige Abnahme des Streulichts und/oder die mittlere optische Weglänge innerhalb des Implantats aus einer Bewertung eines mittleren Brechnungsindex ermittelt wird/werden, wobei die geometrische Wellenlänge bekannt ist.

Eine vorteilhafte Anwendung der Vorrichtung und des Verfahrens besteht darin, dass damit die Blutzuckerkonzentration bestimmt wird, wobei der an sich bekannte Zusammenhang zwischen der Zuckerkonzentration in der Gewebeflüssigkeit und dem Blut genutzt wird.

### Kurze Beschreibung der Zeichnungen

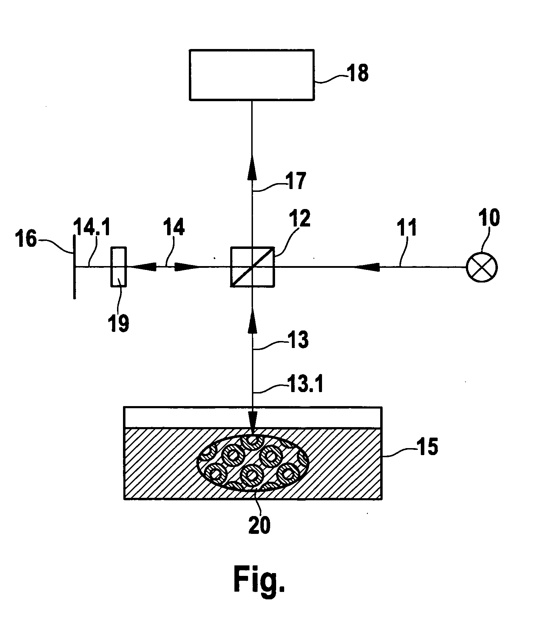

Die Erfindung wird nachfolgend anhand von Ausführungsbeispielen unter Bezugnahme auf die Zeichnung näher erläutert.

### Ausführungsform der Erfindung

Die Fig. zeigt in schematischer Darstellung eine interferometrische Messvorrichtung mit einem Interferometerteil, der einen Objektzweig 13, in den der zu vermessende Hautabschnitt eingebracht ist, und einen Referenzzweig 14 mit einer Referenz 16 aufweist. Von einer Lichtquelle 10 wird ein Eingangsstrahl 11 auf einen Strahlteiler 12 geführt und von diesem in einen über den Objektzweig 13 3 geführten Objektstrahl 13.1 und einen über den Referenzzweig 14 gerührten Referenzstrahl 14.1 aufgeteilt. Der von der Haut 15 zurückgestreute Objektstrahl und der von der Referenz 16 zurückgestreute Referenzstrahl werden überlagert und zur Interferenz gebracht, wozu die optische Weglänge des Referenzzweigs 14 und des Objektzweigs 13 entsprechend angepasst sind. Die interferierende Strahlung wird von einem Bildaufnehmer einer Detektorvorrichtung 18 aufgenommen, und in einer anschließenden Auswerteeinrichtung werden die sich durch die Interferenz ergebenden Intensitätsmodulationen ausgewertet. Zur Kompensation der Dispersion in den Hautschichten ist in den Referenzzweig 14 ein dispersionskompensierendes Medium 19 eingebracht.

Die interferometrische Messvorrichtung dient dem Zweck, tiefenaufgelöst optische Informationen zu ermitteln, insbesondere die in einer bestimmten Tiefe der Hautstruktur zurückgestreute Intensität. Je nach Art des interferometrischen Aufbaus handelt es sich bei der Lichtquelle 10 entweder um eine sogenannte Weißlichtquelle, mit der eine bestimmte Kohärenzlänge verbunden ist, oder um eine schmalbandige, durchstimmbare (Laser-)Lichtquelle.

Das Licht in dem Objektzweig 13 wechselwirkt mit den dort befindlichen Substanzen, wobei als Besonderheit in die Haut ein Implantat eingebracht ist, um eine oder mehrere definierte Grenzflächen auszubilden. Abhängig von der Brechungsindexdifferenz zwischen der an die Grenzflächen des Substrats angrenzenden Hautsubstanz, insbesondere der Gewebeflüssigkeit, und dem Implantatmaterial wird ein Lichtanteil in Richtung Strahlteiler zurückgeworfen.

Das Licht im Referenzzweig 14 trifft auf die reflektierende Referenz 16, z.B. einen ebenen Spiegel, und wird von dort zurückgeworfen. Je nach Menge des im Objektzweig 13 zurückgeworfenen Lichtes kann es auch vorteilhaft sein, den Reflexionsgrad im Referenzzweig 14 durch weitere Maßnahmen geeignet abzustimmen. Zur Tiefenabtastung des zu vermessenden Hautabschnittes kann z.B. die Referenz 16 im Referenzzweig 14 in Richtung des Strahlteilers bewegt werden.

Je nach interferometrischem Aufbau bzw. Verfahren findet der Tiefenscan dadurch statt, dass die Referenz 16 im Referenzzweig 14 bewegt wird oder die Wellenlänge der Lichtquelle 10 definiert verändert wird. Eine weitere Alternative besteht darin, dass die Detektorvorrichtung 18 zur spektralen Zerlegung der erfassten Strahlung ausgebildet ist und der Auswertung die spektral aufgelöste Strahlung zugrunde gelegt wird, wobei eine Umrechnung in den Ortsraum nach an sich bekannten Methoden erfolgt.

Die aus dem Objektzweig 13 und dem Referenzzweig 14 zurück kommende und zusammengeführte, interferierende Strahlung 17 erzeugt in der Detektorvorrichtung 18 ein zur momentanen Lichtintensität proportionales Signal entsprechend der Intensitätsmodulation, wobei die interferometrische Intensitätsmodulation durch den vorstehend genannten Scanmechanismus tiefenabhängig erfolgt. Die Höhe der Intensitätsmodulation zu einer bestimmten Scanposition gibt Auskunft über die Menge des zurückgestreuten Lichtes im Objektzweig in einer bestimmten Tiefe.

Das Implantat 20 wird in die Haut eingebracht, um die dann anschließenden nichtinvasiven optischen Messungen durchzuführen, wobei ein biokompatibles Implantat eingesetzt wird. Bei bekanntem und festem Brechungsindex des Implantats 20 wird nun die Menge des zurückgestreuten Lichts an der jeweiligen Grenzfläche zwischen Implantat 20 und Gewebeflüssigkeit durch die Brechungsindexdifferenz der beiden Medien bestimmt. Ist eine den Brechungsindex der betreffenden Hautsubstanz bestimmende Eigenschaft der Hautsubstanz bekannt, kann durch die ermittelte Brechungsindexdifferenz demnach auf diese Eigenschaft rückgeschlossen werden. Beispielsweise kann auf diese Weise die durch die Blutzuckerkonzentration bedingte Änderung des Brechungsindex der Gewebeflüssigkeit ermittelt werden. Durchläuft das Messlicht eine Vielzahl von Grenzflächen zwischen Implantatmaterial und umgebender Hautsubstanz, insbesondere also Gewebeflüssigkeit, ist dementsprechend eine tiefenabhängige Abnahme des zurückgestreuten Lichtes messbar. Um eine Vielzahl von Grenzflächen zu erhalten, wird vorteilhaft eine Mikrostrukturierung des Implantats 20 mit vielen Kanälen oder Hohlräumen, bzw. ein poröses Material verwendet. Die in einem derartigen Implantat 20 beobachtbare tiefenabhängige Abnahme des Streulichts kann also zur Messung der Brechungsindexänderung und damit z.B. zur Blutzuckerbestimmung verwendet werden.

Für den Fall eines mikrostrukturierten oder porösen Implantats 20 ergibt sich eine weitere Methode zur Messung der Brechungsindexänderung. Das Licht erfährt im Durchgang durch das Implantat 20 einen mittleren Brechungsindex, der sich aus der Mittelung der optischen Weglängen im Implantatsmedium und in der Hautsubstanz, insbesondere Gewebeflüssigkeit ergibt. In dem optischen Tiefenscan wird diese mittlere optische Weglänge bestimmt, wenn aus dem Messsignal Vorder- und Rückseite des Implantats hervorgehen. Die gemessene Differenz zwischen den beiden Punkten der Vorderseite und der Rückseite ergibt die optische Weglänge im Implantat 20. Dabei kann das Implantat 20 auch so geformt, strukturiert oder beschichtet werden, dass Vorder- und Rückseitensignal sich im Messsignal besonders hervorheben. Eine Möglichkeit ist hier z.B. die Beschichtung mit einem Medium mit besonders hohem optischem Brechungsindex.

Da andererseits die geometrische Weglänge im Unterschied zur optischen Weglänge konstant bleibt, kann eine Änderung der gemessenen optischen Weglänge auf eine Brechungsindexänderung der Hautsubstanz bzw. Gewebeflüssigkeit innerhalb des Implantats 20 zurückgeführt werden. Diese wiederum steht in direktem Verhältnis zur zu bestimmenden Eigenschaft, beispielsweise also der Blutzuckerkonzentration. Somit steht eine zweite physikalische Messgröße in derselben Messung zur Verfügung, die eine weitere Aussage, eine Erhöhung der Genauigkeit oder eine Kontrolle des Messergebnisses alternativ oder zusätzlich zu dem erstgenannten Verfahren der tiefenabhängigen Streulichtänderung ergibt.

Mit dem Implantat 20 wird für die optische Messung eine definierte feste Größe vorgegeben, wodurch die Messgenauigkeit und Zuverlässigkeit der Messergebnisse sowie die Messgeschwindigkeit erheblich begünstigt werden.

## Patentansprüche

1. Interferometrische Messvorrichtung zur optischen Messung an Hautstrukturen mit einem Strahlteiler (12) zum Aufteilen eines von einer Lichtquelle (10) ausgesandten Eingangsstrahls (11) in einen Objektstrahl (13.1) und einen Referenzstrahl (14.1), mit einem Objektzweig (13), über den der Objektstrahl (13.1) zu der Haut geleitet und der von den Hautstrukturen zurückgestreute Objektstrahl (13.1) zurückgeführt wird, mit einem Referenzzweig (14), über den der Referenzstrahl (14.1) zu einer Referenz (16) geleitet und von dieser zurückgeführt wird, und mit einer Detektorvorrichtung (18) zur Aufnahme der zusammengeführten und interferierenden Strahlung des zurückgestreuten Objektstrahls (13.1) und des zurückgeführten Referenzstrahls (14.1), zur Auswertung der durch die Interferenz erhaltenen Intensitätsmodulation und zur Bestimmung einer Eigenschaft der Hautstruktur aus der tiefenabhängigen Abnahme des Streulichts, wobei zum Zurückstreuen eines Anteils des in die Haut geleiteten Objektstrahls (13.1) ein Implantat (20) mit einer dem einfallenden Objektstrahl (13.1) zugewandten Vorderseite und einer davon abgewandten Rückseite zur Einbringung in die Haut ausgelegt ist, das gegenüber Hautsubstanz, die die für die Einbringung vorgesehene Stelle umgibt, einen unterschiedlichen Brechungsindex aufweist,
**dadurch gekennzeichnet,**
**dass** das Implantat (20) zur Ausbildung einer Vielzahl von Grenzflächen relativ zur Gewebeflüssigkeit zwischen seiner Vorderseite und seiner Rückseite eine Mikrostrukturierung mit vielen Kanälen oder Hohlräumen aufweist oder aus einem porösen Material gebildet ist und
**dass** die Auswertung in der Weise ausgelegt ist, dass bezüglich des Durchgangs des Objektstrahls (13.1) durch das Implantat (20) auf der Basis der Intensitätsmodulation im Interferenzsignal die tiefenabhängige Abnahme des Streulichts und in derselben Messung die mittlere optische Weglänge im Implantat (20) unter Bewertung eines mittleren Brechungsindex für das Licht beim Durchgang durch das Implantat (20) ermittelt werden und sowohl aus der Abnahme des Streulichts als auch aus der Änderung der optischen Weglänge auf eine Brechungsindexänderung geschlossen und daraus die Eigenschaft der Hautstruktur bestimmt wird.

2. Messvorrichtung nach Anspruch 1,
**dadurch gekennzeichnet,**
**dass** zum Bewirken eines Tiefenscans in der Haut und damit zum Erzeugen der Intensitätsmodulation eine Scanvorrichtung vorgesehen ist, die in der Weise aufgebaut ist, dass die optische Weglänge des Referenzstrahls (14.1) in dem Referenzzweig oder die Wellenlänge der Lichtquelle (10) definiert geändert wird oder dass in der Detektorvorrichtung (18) eine spektrale Auflösung und Auswertung erfolgt, wobei eine Umrechnung in den Ortsraum vorgenommen wird.

3. Messvorrichtung nach einem der vorhergehenden Ansprüche,
**dadurch gekennzeichnet,**
**dass** das Implantat (20) so geformt, strukturiert oder beschichtet ist, dass das von seiner Vorderseite herrührende Vorderseitensignal und das von seiner Rückseite herrührende Rückseitensignal besonders hervorgehoben sind.

4. Messvorrichtung nach einem der vorhergehenden Ansprüche,
**dadurch gekennzeichnet,**
**dass** im Referenzzweig (14) ein auf den Durchgangsabschnitt der Haut abgestimmtes dispersionskompensierendes Medium (19) eingebracht ist.

5. Interferometrisches Verfahren zur optischen Messung an einer Hautstruktur, bei dem in die Haut ein Implantat (20) mit gegenüber der umgebenden Hautsubstanz unterschiedlichem Brechungsindex eingebracht ist und ein von einer Lichtquelle (10) kommender Eingangsstrahl (11) in einen zu einer Referenz (16) geführten Referenzstrahl (14.1) und einen zu der Haut (15) geführten Objektstrahl (13.1) aufgeteilt wird, der von der Referenz (16) und von der Haut (15) zurückgestreute Referenzstrahl (14.1) und Objektstrahl (13.1) zur Interferenz gebracht werden und die durch die Interferenz erhaltene Intensitätsmodulation detektiert und zum Bestimmen einer Eigenschaft der Hautstrukturen ausgewertet wird, wobei die durch das an mindestens einer Grenzfläche des Implantats (20) relativ zu der Gewebeflüssigkeit zurückgestreute Licht bewirkte Intensitätsmodulation zum Bestimmen der Eigenschaft der Hautstrukturen ausgewertet wird und zum Bestimmen der Eigenschaft der Hautstrukturen die tiefenabhängige Abnahme des Streulichts ermittelt wird,
**dadurch gekennzeichnet,**
**dass** die tiefenabhängige Abnahme des Streulichts auf der Basis der Intensitätsmodulation ermittelt wird und
**dass** in derselben Messung zusätzlich die mittlere optische Weglänge innerhalb des Implantats (20) aus einer Bewertung eines mittleren Brechnungsindex ermittelt wird, wobei die geometrische Weglänge als bekannt zugrunde gelegt wird.

6. Anwendung der Vorrichtung nach einem der Ansprüche 1 bis 4 oder des Verfahrens nach Anspruch 5 zum Bestimmen der Blutzuckerkonzentration.

## Claims

1. Interferometric measuring device for taking optical measurements at skin structures, with a beam splitter (12) for splitting an input beam (11) emitted by a light source (10) into an object beam (13.1) and a reference beam (14.1), with an object branch (13), by means of which the object beam (13.1) is guided to the skin and the object beam (13.1) backscattered from the skin structures is returned, with a reference branch (14), by means of which the reference beam (14.1) is guided to a reference (16) and returned therefrom, and with a detector device (18) for recording the combined and interfering radiation of the backscattered object beam (13.1) and of the returned reference beam (14.1), for evaluating the intensity modulation obtained by the interference and for determining a property of the skin structure from the depth-dependent reduction in the scattered light, an implant (20) with a front side facing the incident object beam (13.1) and a rear side facing away from it being designed to be inserted into the skin for backscattering a portion of the object beam (13.1) guided into the skin, which implant has a different refractive index to the skin substance that surrounds the site intended for the insertion,
**characterized**
**in that** the implant (20) has microstructuring between its front side and its rear side with many channels or cavities or is formed from a porous material in order to form a multiplicity of boundary surfaces relative to the tissue fluid and in that the evaluation is designed such that the depth-dependent reduction in the scattered light and, during the same measurement, the mean optical path length in the implant (20) are established in respect of the passage of the object beam (13.1) through the implant (20) on the basis of the intensity modulation in the interference signal during the evaluation of an average refractive index of the light passing through the implant (20), and a change in refractive index is deduced from both the reduction in scattered light and the change in the optical path length, and this is used to determine the property of the skin structure.

2. Measuring device according to Claim 1,
**characterized**
**in that** a scan device is provided for bringing about a depth scan in the skin and thus generating the intensity modulation, said scan device being designed such that there is a defined change in the optical path length of the reference beam (14.1) in the reference branch or in the wavelength from the light source (10), or in that there is spectral resolution and evaluation in the detector device (18), with a conversion into real space being undertaken.

3. Measuring device according to one of the preceding claims,
**characterized**
**in that** the implant (20) is formed, structured or coated such that the front-side signal originating from its front side and the rear-side signal originating from its rear side are particularly accentuated.

4. Measuring device according to one of the preceding claims,
**characterized**
**in that** a dispersion-compensating medium (19) matched to the passage section of the skin is introduced into the reference branch (14).

5. Interferometric method for taking optical measurements at a skin structure, in which an implant (20) with a refractive index different from the surrounding skin substance has been inserted into the skin and an input beam (11) originating from a light source (10) is divided into a reference beam (14.1) guided to a reference (16) and an object beam (13.1) guided to the skin (15), the reference beam (14.1) and the object beam (13.1) backscattered from the reference (16) and the skin (15) being brought into interference and the intensity modulation obtained by the interference being detected and being evaluated to determine a property of the skin structures, with the intensity modulation brought about by the light backscattered at at least one boundary surface of the implant (20) relative to the tissue fluid being evaluated to determine the property of the skin structures and the depth-dependent reduction in the scatted light being established to determine the property of the skin structures,
**characterized**
**in that** the depth-dependent reduction in the scattered light is established on the basis of the intensity modulation and
**in that** the mean optical path length within the implant (20) is also established during the same measurement from evaluating a mean refractive index, with the assumption being made that the geometric path length is known.

6. Application of the device according to one of Claims 1 to 4 or the method according to Claim 5 for determining the blood-sugar concentration.

## Revendications

1. Dispositif de mesure interférométrique pour la mesure optique sur des structures dermiques, comprenant un diviseur de rayon (12) pour diviser un rayon d'entrée (11) émis par une source de lumière (10) en un rayon d'objet (13.1) et un rayon de référence (14.1), comprenant une branche d'objet (13) par le biais de laquelle le rayon d'objet (13.1) est guidé vers la peau et le rayon d'objet (13.1) rétrodiffusé par les structures dermiques est renvoyé, comprenant une branche de référence (14) par le biais de laquelle le rayon de référence (14.1) est guidé vers une référence (16) et renvoyé depuis celle-ci, et comprenant un dispositif de détection (18) pour accueillir le rayonnement rassemblé et mettre en interférence le rayon d'objet (13.1) rétrodiffusé et le rayon de référence (14.1) renvoyé, pour interpréter la modulation d'intensité obtenue par l'interférence et pour déterminer une propriété de la structure dermique à partir de la diminution de la lumière diffusée en fonction de la profondeur, un implant (20) ayant un côté avant qui fait face au rayon d'objet (13.1) incident et un côté arrière à l'opposé de celui-ci étant conçu pour être implanté dans la peau pour rétrodiffuser une partie du rayon d'objet (13.1) guidé dans la peau, lequel présente un indice de réfraction différent de celui de la substance dermique qui entoure l'endroit prévu pour l'implantation,
**caractérisé en ce**
**que** l'implant (20), pour former une pluralité de surfaces de délimitation par rapport au liquide structurel, présente entre son côté avant et son côté arrière une microstructure incluant de nombreux canaux ou espaces creux ou est constitué d'un matériau poreux et
**que** l'interprétation est conçue de telle sorte que concernant le passage du rayon d'objet (13.1) à travers l'implant (20), la diminution de la lumière diffusée en fonction de la profondeur est établie en se basant sur la modulation d'intensité dans le signal d'interférence et, dans la même mesure, la longueur moyenne du trajet optique dans l'implant (20) est établie en évaluant un indice de réfraction moyen pour la lumière lors du passage à travers l'implant (20) et une modification de l'indice de réfraction est déduite à la fois de la diminution de la lumière diffusée et de la modification de la longueur du trajet optique et la propriété de la structure dermique est déterminée à partir de cela.

2. Dispositif de mesure selon la revendication 1, **caractérisé en ce que** pour réaliser un balayage en profondeur dans la peau et générer ainsi la modulation d'intensité, il est prévu un dispositif de balayage qui est construit de telle sorte que la longueur du trajet optique du rayon de référence (14.1) dans la branche de référence ou la longueur d'onde de la source de lumière (10) est modifiée de manière définie ou qu'une résolution et une interprétation spectrales ont lieu dans le dispositif de détection (18), une conversion dans l'espace local étant effectuée.

3. Dispositif de mesure selon l'une des revendications précédentes, **caractérisé en ce que** l'implant (20) est façonné, structuré ou recouvert d'un revêtement de telle sorte que le signal de côté avant provenant de son côté avant et le signal de côté arrière provenant de son côté arrière sont particulièrement mis en valeur.

4. Dispositif de mesure selon l'une des revendications précédentes, **caractérisé en ce qu'**un milieu (19) de compensation de la dispersion adapté à la section de traversée de la peau est incorporé dans la branche de référence (14).

5. Procédé interférométrique pour la mesure optique sur une structure dermique, avec lequel un implant (20) ayant un indice de réfraction différent de celui de la substance dermique environnante est implanté dans la peau et un rayon d'entrée (11) provenant d'une source de lumière (10) est divisé en un rayon de référence (14.1) guidé vers une référence (16) et un rayon d'objet (13.1) guidé vers la peau (15), le rayon de référence (14.1) et le rayon d'objet (13.1) rétrodiffusés par la référence (16) et par la peau (15) sont amenés en interférence et la modulation d'intensité obtenue par l'interférence est détectée puis interprétée pour déterminer une propriété des structures dermiques, la modulation d'intensité provoquée par la lumière rétrodiffusée au niveau d'au moins une surface de délimitation de l'implant (20) par rapport au liquide structurel étant interprétée pour déterminer la propriété des structures dermiques et pour déterminer la propriété des structures dermiques, la diminution de la lumière diffusée en fonction de la profondeur étant établie,
**caractérisé en ce**
**que** la diminution de la lumière diffusée en fonction de la profondeur est établie en se basant sur la modulation d'intensité et
**que** la longueur moyenne du trajet optique à l'intérieur de l'implant (20) est établie en outre au cours de la même mesure à partir d'une évaluation d'un indice de réfraction moyen, la longueur géométrique du trajet étant supposée connue.

6. Utilisation du dispositif selon l'une des revendications 1 à 4 ou du procédé selon la revendication 5 pour déterminer le taux de glycémie dans le sang.
